# EUROPÄISCHE PATENTSCHRIFT

(11) **EP 1 005 319 B1**
(45) Veröffentlichungstag und Bekanntmachung des Hinweises auf die Patenterteilung: **17.03.2004**
(21) Anmeldenummer: 98949963.7
(22) Anmeldetag: 21.08.1998
(51) Int. Cl.: A61K 6/093, A61K 6/10, C08L 83/04, C08K 5/54

(54) **HÄRTBARE MASSE MIT SILAN-DENDRIMEREN**
HARDENABLE MASS WITH SILANE DENDRIMERS
MATIERE DURCISSABLE CONTENANT DES DENDRIMERES DE SILANE

(30) Priorität: 22.08.1997 DE 19736665
(43) Veröffentlichungstag der Anmeldung: 07.06.2000
(73) Patentinhaber: 3M ESPE AG, 82229 Seefeld (DE)
(72) Erfinder: ZECH, Joachim, D-82229 Hechendorf (DE); LECHNER, Günther, D-82237 Wörthsee (DE)
(74) Vertreter: Freiherr von Wittgenstein, Arved, Dr.
(86) Internationale Anmeldenummer: PCT/EP1998/005319
(87) Internationale Veröffentlichungsnummer: WO 1999/009934

(56) Entgegenhaltungen:
- EP-A- 0 743 313
- DE-A- 19 517 838

## Beschreibung

Die Erfindung betrifft härtbare Zusammensetzungen, die hochverzweigte Moleküle auf Silanbasis enthalten und sich besonders als Dentalmaterialien eignen.

Bei Dentalmaterialien besteht für die verschiedensten Indikationen ein starker Bedarf an Massen mit hoher Endhärte. Als Beispiele können Massen für die Bißregistrierung, provisorische und permanente Füllungsmaterialien, Kronen- und Brückenmaterialien sowie Zemente und Lacke genannt werden. Die Endhärte spielt bei all diesen Anwendungszwecken eine wichtige Rolle, beispielsweise bestimmt sie die Formstabilität, Beschneidbarkeit und Ausgießbarkeit von Abdruckmassen.

Üblicherweise werden zur Erhöhung der Endhärte von härtbaren Massen der Füllstoffgehalt der Massen oder der Funktionalitätsgrad der reaktiven Monomeren erhöht oder die Kettenlänge der reaktiven Monomeren erniedrigt.

Polymerisierbare Zusammensetzungen, die hohe Füllstoffgehalte aufweisen und sich als Dentalmaterialien eignen, sind beispielsweise in der International Encyclopedia of Composites (S. M. Lee, Herausgeber, Vol. 2, VCH-Verlagsgesellschaft, New York 1990, Seite 182) und von L. Ehrnford (Swed. Dent. J. Suppl. 18, 1983) beschrieben. Aus der EP-A-0 480 472 sind hochgefüllte Massen bekannt, die jedoch nur unter Einwirkung von Ultraschall-Schwingungen verarbeitbar sind.

Bekannt sind ferner aus der DE-A-2 646 726 kurzkettige vinylendgestoppte Polydimethylsiloxane der allgemeinen Formel:

CH₂=CH-R₂SiO-(SiR₂O)ₙ-SiR₂-CH=CH₂

worin R gleiche oder verschiedene, von aliphatischen Mehrfachbindungen freie, einwertige, gegebenenfalls substituierte Kohlenwasserstoffreste und n 0 oder eine ganze Zahl von 1 bis 6 bedeuten. Diese Verbindungen werden als Inhibitoren zum Regeln der Vernetzungsgeschwindigkeit von additionsvernetzenden Silikonabformmassen eingesetzt.

Die DE-A-4 122 310 offenbart hingegen, daß Verbindungen der gleichen allgemeinen Formel, worin jedoch n eine ganze Zahl von 10 bis 20 bedeutet, keineswegs inhibierend wirken, sondern zur Erhöhung der Endhärte der vernetzten Gummis eingesetzt werden können.

Diesen Schriften ist somit zu entnehmen, daß zwischen zwei terminalen C-C-Doppelbindungen in Molekülen der angegebenen allgemeinen Formel mindestens 22 Kettenglieder liegen müssen, um eine Härteerhöhung des Gummis zu erzielen; bei geringeren Abständen zwischen den C-C-Doppelbindungen erfolgt eine Inhibierung der Vernetzung. Dieser Sachverhalt ist außerdem aus der DE-A-4 324 685 bekannt, in der als Beispiele für Polymerisationsinhibitoren u.a. Diallylmaleinat und 1,3-Divinyl-1,1,3,3-tetramethyldisiloxan genannt werden. Gleichfalls werden solchen Molekülen mit mehr als 42 Kettengliedern zwischen den terminalen Doppelbindungen keine vernetzenden Eigenschaften zugeschrieben.

Es besteht ein hoher Bedarf an vernetzenden Monomeren, die die Endhärte einer härtbaren Masse erhöhen, aber die übrigen Eigenschaften, insbesondere die Viskosität der Masse nicht negativ beeinflussen.

Aufgabe der vorliegenden Erfindung ist es, härtbare Massen mit erhöhter Endhärte und zur Verarbeitung auf dentalem Gebiet geeigneter Viskosität bereitzustellen.

Die Aufgabe wird gelöst durch eine härtebare Masse, enthaltend:
(A) gegebenenfalls Organopolysiloxane mit mindestens zwei ungesättigten Gruppen im Molekül,
(B) Organohydrogenpolysiloxane mit mindestens 3 SiH-Gruppen im Molekül,
(C) gegebenenfalls Organopolysiloxane ohne reaktive Gruppen,
(D) Katalysator,
(E) gegebenenfalls Hydrophilierungsmittel,
(F) Füllstoffe und
(G) gegebenenfalls übliche dentale Zusatz-, Hilfs- und Farbstoffe,
   dadurch gekennzeichnet, daß sie zusätzlich
(H) mindestens ein Silan-Dendrimer mit terminalen Alkenylgruppen enthält.

Überraschenderweise wurde festgestellt, daß ein Zusatz von Silan-Dendrimeren zu härtbaren Massen die Endhärte dieser Massen stark erhöht, aber gleichzeitig der Viskositätserhöhung der nicht-gehärteten Massen entgegenwirkt, obwohl die Silan-Dendrimeren mindestens in der ersten Generation einen deutlich geringeren Doppelbindungsabstand aufweisen als dies im Stand der Technik als zwingend erforderlich angesehen wurde, um keine inhibierende Wirkung zu zeigen. Ferner ist bei benachbarten Doppelbindungen stets die Gefahr der Komplexbildung am Katalysator gegeben und daher auch eine inhibierende Wirkung zu beobachten. Um so erstaunlicher ist das Gelingen einer Polymerisation unter Verwendung der erfindungsgemäßen Silan-Dendrimeren.

Bevorzugt sind als Komponente (A) Diorganopolysiloxane mit terminalen Triorganosiloxygruppen, von denen mindestens eine der drei organischen Gruppen eine Vinylgruppe ist. Bevorzugte Diorganosiloxane dieser Struktur werden durch folgende Formel wiedergegeben: 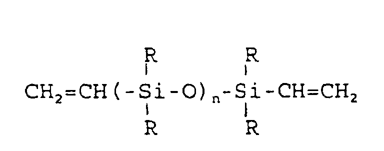 in der R eine unsubstituierte oder substituierte monovalente Kohlenwasserstoffgruppe mit 1 bis 6 C-Atomen repräsentiert, die bevorzugt frei von aliphatischen Mehrfachbindungen ist und n so gewählt ist, daß die Viskosität zwischen 4 und 50.000 mPa·s liegt. Mindestens 50 % der Reste R sind bevorzugt Methylgruppen und Beispiele für andere Gruppen R sind Ethyl-, Vinyl- und 3,3,3-Trifluorpropylgruppen. Derartige Moleküle sind in der US-A-4 035 453 beschrieben, deren Offenbarung hierin soweit mit umfaßt sein soll. Die Herstellung der Komponente (A) erfolgt nach üblichen Verfahren, die z.B. in W.

Noll, "Chemie und Technologie der Silikone", Verlag Chemie Weinheim, 2. Auflage 1964, Seite 162-206 oder J. Burghardt, Chemie und Technologie der Polysiloxane in "Silikone, Chemie und Technologie", Vulkan Verlag, Essen, 1989, Seiten 23-37 dargestellt sind.

Besonders bevorzugt sind lineare Polydimethylsiloxane obiger Struktur mit den angegebenen Viskositätsbereichen, bei denen die Endgruppen aus Dimethylvinylsiloxyeinheiten bestehen und die weiteren Substituenten R in der Kette aus Methylgruppen bestehen.

Komponente (B) ist bevorzugt ein Organopolysiloxan mit mindestens 3 Si-gebundenen Wasserstoffatomen pro Molekül. Dieses Organopolysiloxan enthält bevorzugt von 0,01 bis 1,7 Gew.-% siliciumgebundenen Wasserstoff. Die Silikonvalenzen, die nicht mit Wasserstoff- oder Sauerstoffatomen abgesättigt sind, werden mit monovalenten Kohlenwasserstoffradikalen abgesättigt, die frei von aliphatischen Mehrfachbindungen sind. Die Kohlenwasserstoffradikale können substituiert oder unsubstituiert sein. Mindestens 50 %, bevorzugt 100 %, der Kohlenwasserstoffradikale, die an Siliciumatome gebunden sind, bestehen aus Methylradikalen. Auch derartige Komponenten sind in den oben genannten Literaturstellen hinsichtlich Struktur und Herstellung beschrieben.

Die Mengenverhältnisse der Komponenten (A), (B) und (H) sind bevorzugt derart gewählt, daß pro Mol an ungesättigter Doppelbindung der Komponenten (A) und (H) 0,5 bis 10 Mol SiH-Einheiten aus der Komponente (B) vorliegen. Die Summe der Komponenten (A), (H) und der Komponente (B) liegen im Bereich von 5 bis 70 Gew.-%, bezogen auf das Gesamtgewicht aller Komponenten. Bevorzugt liegen sie im Bereich von 10 bis 60 Gew.-% und insbesondere in einem Bereich von 15 bis 50 Gew.-%.

Geeignete Komponenten (C) sind polymere Organosiloxane ohne reaktive Substituenten, wie sie z.B. in W. Noll "Chemie und Technologie der Silikone", Verlag Chemie Weinheim, 1968, Seite 212 ff. beschrieben sind. Es handelt sich bevorzugt um lineare, verzweigte oder cyclische Organopolysiloxane, bei denen sämtliche Siliciumatome von Sauerstoffatomen oder monovalenten Kohlenwasserstoffradikalen umgeben sind, wobei die Kohlenwasserstoffradikale substituiert oder unsubstituiert sein können. Die Kohlenwasserstoffradikale können Methyl, Ethyl, C₂-C₁₀-Aliphaten, Trifluorpropylgruppen, sowie aromatische C₆-C₁₂-Substituenten sein. Die Komponente (c) trägt zur Verdünnung und Aufweitung des Gumminetzwerkes bei und wirkt als Weichmacher für das ausgehärtete Material. Als relativ günstige Komponente trägt sie auch bei zur Reduktion der Herstellungskosten der erfindungsgemäßen Dentalmassen.

Besonders bevorzugt als Komponente (C) sind Polydimethylsiloxane, welche Trimethylsiloxy-Endgruppen aufweisen. Die Menge an Komponente (C) beträgt 0 bis 40 Gew.-%, bevorzugt 0 bis 20 Gew.-%, besonders bevorzugt 0,1 bis 10 Gew.-%.

Komponente (D) ist bevorzugt ein Platinkomplex, der aus Hexachlorplatinsäure durch Reduktion mit Tetramethyldivinyldisiloxan hergestellt wurde. Diese Verbindungen sind an sich bekannt. Geeignet sind auch andere Platinverbindungen, die die Additionsvernetzung beschleunigen. Gut geeignet sind z.B. Platin-Siloxan-Komplexe, wie sie z.B. in den US-A-3 715 334, US-A-3 775 352 und US-A-3 814 730 beschrieben sind. Der Platinkatalysator wird vorzugsweise in Mengen von 0,00005 bis 0,05 Gew.-%, insbesondere 0,0002 bis 0,04 Gew.-%, jeweils berechnet als elementares Platin und bezogen auf das Gesamtgewicht der mit den Komponenten (A) bis (H) vorliegenden Masse, eingesetzt. Zur Steuerung der Reaktivität kann es notwendig sein, daß ein Inhibitor zugesetzt werden muß, der die vorzeitige Vernetzung zum Elastomeren behindert. Derartige Inhibitoren sind bekannt und z.B. in US-A-3 933 880 beschrieben. Beispiele hierfür sind acetylenisch ungesättigte Alkohole, wie 3-Methyl-1-butin-3-ol, 1-Ethinylcyclohexan-1-ol, 3,5-Dimethyl-1-hexin-3-ol und 3-Methyl-1-pentin-3-ol. Beispiele für Inhibitoren auf Vinylsiloxanbasis sind 1,1,3,3-Tetramethyl-1,3-divinylsiloxan und vinylgruppenhaltige Poly-, Oligo- und Disiloxane.

Komponente (E) ist ein eine hydrophile Natur verleihendes Mittel oder Hydrophilierungsmittel, das den Randwinkel eines Tropfens Wasser oder wäßriger Zusammensetzung (z.B. Gipssuspension, etc.) gegenüber der Silikonzusammensetzung erniedrigt und damit eine bessere Benetzbarkeit der Gesamtzusammensetzung im feuchten Mundmilieu und damit ein besseres Anfließverhalten der Pasten hervorruft. Die Messung des Randwinkels zur Bestimmung der Hydrophilie von Abformmassen ist z.B. in der DE-A-43 06 997, Seite 5, beschrieben, wobei hierauf Bezug genommen wird. Die Hydrophilierungsmittel sind bevorzugt nicht mit reaktiven Gruppen versehen, so daß kein Einbau in das Polysiloxannetzwerk stattfindet. Geeignete Hydrophilierungsmittel sind bevorzugt nicht einbaubare Benetzungsmittel aus der Gruppe der hydrophilen Silikonöle, die in WO 87/03001 und in EP-B-0 231 420 beschrieben sind, auf deren Offenbarung hier insoweit Bezug genommen werden soll. Bevorzugt sind ferner die ethoxylierten Fettalkohole, die in EP-B-0 480 238 beschrieben sind. Desweiteren sind bevorzugte Hydrophilierungsmittel die aus WO 96/08230 bekannten Polyethercarbosilane. Bevorzugt sind auch die in WO 87/03001 beschriebenen nicht-ionischen perfluoralkylierten oberflächenaktiven Substanzen. Ebenfalls bevorzugt sind die nicht-ionischen oberflächenaktiven Substanzen, die in EP-B-0 268 347 beschrieben sind, d.h. die darin aufgeführten Nonylphenolethoxylate, Polyethylenglykol-mono- und -diester, Sorbitanester, sowie Polyethylenglykol-mono- und -diether. Die Einsatzmengen der Hydrophilierungsmittel betragen 0 bis 10 Gew.-%, bezogen auf das Gesamtgewicht aller Komponenten, bevorzugt 0 bis 2 Gew.-% und besonders bevorzugt 0,2 bis 1 Gew.-%. Der nach 3 Min. gemessene Randwinkel eines Wassertropfens auf der Oberfläche eines ausgehärteten erfindungsgemäßen Materials beträgt bevorzugt weniger als 60°, besonders bevorzugt < 50°, insbesondere < 40°.

Zu den einsetzbaren Füllstoffen gemäß Komponente (F) gehören nicht verstärkende Füllstoffe, wie Quarz, Christobalit, Calciumsilikat, Diatomeenerde, Zirkoniumsilikat, Montmorillonite, wie Benthonite, Zheolite, einschließlich der Molekularsiebe, wie Natriumaluminiumsilikat, Metalloxidpulver, wie Aluminiumoder Zinkoxide bzw. deren Mischoxide, Bariumsulfat, Calciumcarbonat, Gips, Glas- und Kunststoffpulver. Zu möglichen Füllstoffen gehören auch verstärkende Füllstoffe, wie z.B. pyrogene oder gefällte Kieselsäure und Siliciumaluminiummischoxide. Die genannten Füllstoffe können hydrophobiert sein, beispielsweise durch die Behandlung mit Organosilanen bzw. -siloxanen oder durch die Veretherung von Hydroxylgruppen zu Alkoxygruppen. Es kann eine Art von Füllstoff, es kann auch ein Gemisch von mindestens zwei Füllstoffen eingesetzt werden. Die Kornverteilung wird vorzugsweise so gewählt, daß keine Füllstoffe mit Korngrößen > 50 µm enthalten sind. Der Gesamtgehalt der Füllstoffe (F) liegt im Bereich von 10 bis 90 %, bevorzugt 30 bis 80 %.

Besonders bevorzugt ist eine Kombination aus verstärkenden und nicht verstärkenden Füllstoffen. Hierbei liegen die verstärkenden Füllstoffe in Mengenbereichen von 1 bis 10 Gew.-%, insbesondere 2 bis 5 Gew.-%. Die Differenz zu den genannten Gesamtbereichen, also 9 bis 70 Gew.-%, insbesondere 28 bis 55 Gew.-%, bilden die nicht verstärkenden Füllstoffe.

Bevorzugt als verstärkende Füllstoffe sind pyrogen hergestellte hochdisperse Kieselsäuren, die bevorzugt durch Oberflächenbehandlung hydrophobiert worden sind. Die Oberflächenbehandlung kann beispielsweise mit Dimethyldichlorsilan, Hexamethyldisilasan, Tetramethylcyclotetrasiloxan oder Polymethylsiloxanen erfolgen.

Besonders bevorzugte nichtverstärkende Füllstoffe sind Quarze, Christobalite und Natriumaluminiumsilikate, die oberflächenbehandelt sein können. Die Oberflächenbehandlung kann prinzipiell mit den gleichen Methoden erfolgen wie im Fall der verstärkenden Füllstoffe beschrieben.

Weiterhin können die erfindungsgemäßen Dentalmassen als Komponente (G) gegebenenfalls Zusätze wie Weichmacher, Pigmente, Antioxidationsmittel, Trennmittel, u.ä. enthalten. Ebenso können auch beispielsweise fein verteiltes Palladium oder Platin als Wasserstoffabsorber enthalten sein. Die Metalle können dabei auch auf Trägermaterialien aufgebracht sein. Die erfindungsgemäßen Massen enthalten derlei Zusatzstoffe in Mengen von vorzugsweise 0 bis 2 Gew.-%, besonders bevorzugt von 0 bis 1 Gew.-%.

Die Komponente (H) besteht aus Silan-Dendrimeren. Allgemein werden als Dendrimere dreidimensionale, hochgeordnete oligomere und polymere Verbindungen bezeichnet, die ausgehend von kleinen Kernmolekülen durch eine sich ständig wiederholende Reaktionsfolge synthetisiert werden. Als Kernmolekül eignen sich monomere oder polymere Moleküle mit mindestens einer reaktiven Stelle. Diese wird in einer ein- oder mehrstufigen Reaktion mit einem Reaktanten umgesetzt, der sich an die reaktive Stelle des Kernmoleküls addiert und der seinerseits mindestens zwei neue reaktive Stellen mitbringt. Die Umsetzung von Kernmolekül und Reaktant ergibt die Kernzelle (Generation Null). Durch eine Wiederholung der Reaktion werden die reaktiven Stellen der ersten Reaktantenschicht mit weiteren Reaktanten umgesetzt, wobei wieder jeweils mindestens zwei neue Verzweigungsstellen ins Molekül eingebracht werden (1. Generation). Die fortschreitende Verzweigung führt zu einem geometrischen Wachstum der Anzahl der Atome für jede Generation. Da die Gesamtgröße wegen der durch die Reaktanten festgelegten Zahl der möglichen kovalenten Bindungen nur linear wachsen kann, werden die Moleküle von Generation zu Generation gedrängter, und sie verändern ihre Form von seesternartig zu kugelig.

Erfindungsgemäß als Komponente (H) einzusetzende Dendrimere können Dendrimere der nullten und jeder weiteren Generation sein. Bevorzugt sind hierbei solche der nullten und ersten Generation, obwohl auch solche weit höherer Generation eingesetzt werden können.

Die Dendrimere der nullten Generation entsprechen der allgemeinen Formel:

SiRₙR'₄₋ₙ

in welcher bedeuten:
R eine Alkenylgruppe mit 2 bis 5 C-Atomen, wobei die C-C-Doppelbindung terminal vorliegt, vorzugsweise eine Vinyl- oder Allylgruppe,
n eine ganze Zahl von 1 bis 4, vorzugsweise = 4 und
R' einen verzweigten oder unverzweigten aliphatischen oder aromatischen Kohlenwasserstoff mit 1 bis 10 C-Atomen oder Wasserstoff, vorzugsweise Methyl oder Phenyl.

Dendrimere anderer Generationen werden durch Umsetzung von Tri- oder Tetraalkenylsilanen (bevorzugt Allyl und Vinyl) als Kernmolekül in einer ersten Stufe mit Hydrogen-Chlor-Silanen erhalten. Diese Produkte werden in einer weiteren Stufe mit Alkenyl-Grignard-Verbindungen umgesetzt.

Besonders bevorzugt sind hierbei Dendrimere der ersten Generation folgender Formel:

SiR'ₓ((CH₂)ₙ-Si-((CH₂)ₘ-CH=CH₂)₃)₄₋ₓ

in welcher bedeuten:
R' wie oben definiert,
n = 2, 3, 4 oder 5,
m = 0, 1, 2 oder 3, und
x = 0 oder 1.

Besonders bevorzugte Dendrimere nach dieser allgemeinen Formel sind:
Me-Si((CH₂-CH₂-Si(Vinyl)₃)₃
Si((CH₂-CH₂-Si(Vinyl)₃)₄
Me-Si((CH₂-CH₂-CH₂-Si(Allyl)₃)₃
Si((CH₂-CH₂-CH₂-Si(Allyl)₃)₄
Me-Si((CH₂-CH₂-Si(Allyl)₃)₃
Si((CH₂-CH₂-Si(Allyl)₃)₄
Me-Si((CH₂-CH₂-CH₂-Si(Vinyl)₃)₃
Si((CH₂-CH₂-CH₂-Si(Vinyl)₃)₄

A. W. van der Made und P.W.N.M. van Leeuwen beschreiben in J. Chem. Soc. Commen. (1992), Seite 1400 und in Adv. Mater. (1993), 5, No. 6, Seite 366 ff. die prinzipielle Synthese dieser Silandendrimere. Die Synthese beginnt beispielsweise mit vollständiger Allylierung von Tetrachlorsilan mittels 10 % Überschuß an Allylmagnesiumbromid in Diethylether zu Tetraallylsilan. Daraufhin werden die Allylgruppen mit Trichlorsilan in Gegenwart eines Platinkatalysators hydrosilyliert. Zuletzt erfolgt die Umsetzung mit Allylmagnesiumbromid in Diethylether. Als Ergebnis wird ein Dendrimer mit 12 Allylendgruppen erhalten. Diese erste Generation kann auch zu einer zweiten Generation umgesetzt werden, wobei 36 Allylgruppen erhalten werden. Die gleiche Thematik wird auch von D. Seyferth und D.Y. Son in Organometallics (1994), 13, 2682-2690 behandelt.

Möglich sind ferner Umsetzungsprodukte von Tri- bzw. Tetra- bzw. Penta- bzw. Hexa- bzw. Hepta- bzw. Octaalkenyl(cyclo)siloxanen mit Hydrogen-Chlor-Silanen als Kernmolekül. Diese werden in einer weiteren Stufe mit Alkenyl-Grignard-Verbindungen umgesetzt und führen zu Dendrimeren mit cyclischen oder linearen Siloxankernen, die den folgenden allgemeinen Formeln entsprechen: 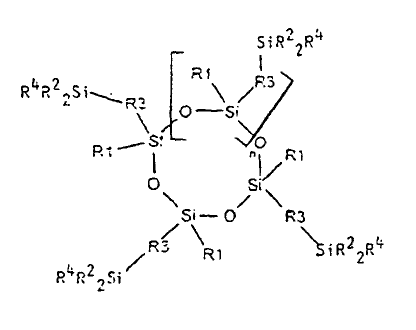 in welcher bedeuten:
- n =: 0, 1, 2, 3, 4 oder 5,
- R¹: einen gesättigten Alkylrest mit 1 bis 6 C-Atomen oder Wasserstoff,
- R²: eine Alkenylgruppe mit 2 bis 6 C-Atomen, wobei die C-C-Doppelbindung terminal vorliegt, vorzugsweise eine Vinyloder Allylgruppe,
- R³: eine Alkylengruppe mit 2 bis 6 C-Atomen und
- R⁴: entweder R² oder -O-SiMe₂-Allyl oder -O-SiMe₂-Vinyl.

Bevorzugt sind hierbei cyclische Vertreter mit n = 0 oder 1, R¹ = CH₃ und R² = Allyl oder Vinyl.

Geeignet sind auch Dendrimere höherer Generationen als durch die obigen Formeln zum Ausdruck kommt.

Erfindungsgemäß können sowohl gereinigte Tri-, Tetra-, Penta-, Hexa-, Hepta- oder Octasiloxan-Dendrimere verwendet werden, als auch beliebige Gemische dieser Dendrimere.

Silan-Dendrimere, deren Herstellung und Verwendung bei Lacken sind auch aus den DE-A-196 03 242 und 195 17 838 sowie aus der EP-A-0 743 313 bekannt. Dort angeführte Dendrimere eignen sich ebenso für den erfindungsgemäßen Zweck.

Geeignet sind ferner polyfunktionelle Alkenylverbindungen als Kerne. Insbesondere geeignet sind Trimethylolpropantriallylether, Tetraallylpentaerythrit, Santolink XI-100 (Monsanto), Tetraallyloxyethan, 1,3,5-Benzoltricarbonsäuretriallylester, 1,2,4-Benzoltricarbonsäuretriallylester, 1,2,4,5-Benzoltetracarbonsäuretetraallylester, Triallylphosphat, Triallylcitrat, Triallylisocyanurat, Triallyloxytriazin, Hexallylinosit, sowie ganz allgemein Verbindungen, die wenigstens zwei ethylenisch ungesättigte Gruppen besitzen, die gegebenenfalls substituiert sein können, beispielsweise O-Allyl-, N-Allyl-, O-Vinyl-, N-Vinyl-, p-Vinylphenolether-Gruppen. Mögliche Polyene sind auch in US-A-3 661 744 und EP-A-0 188 880 beschrieben. Das Polyen kann z.B. folgende Struktur aufweisen: (Y)-(X)ₘ, wobei m eine ganze Zahl größer oder gleich 2, vorzugsweise 2, 3 oder 4 ist, und X ausgewählt ist aus der Gruppe - [RCR]_{f}CR=CRR, -O-CR=CR-R, -S-CR=CR-R, -NR-CR=CR-R, worin f eine ganze Zahl von 1 bis 9 ist und die Reste R die Bedeutungen H, F, Cl, Furyl, Thienyl, Pyridyl, Phenyl und substituiertes Phenyl, Benzyl und substituiertes Benzyl, Alkyl und substituiertes Alkyl, Alkoxy und substituiertes Alkoxy sowie Cycloalkyl und substituiertes Cycloalkyl besitzen und jeweils gleich oder verschieden sein können. (Y) ist ein mindestens difunktioneller, organischer Rest, der aus Atomen aufgebaut ist, die ausgewählt sind aus der Gruppe C, O, N, Cl, Br, F, P, Si und H.

Gut geeignete Polyen-Verbindungen sind beispielsweise die Allyl- und/oder Vinylester mindestens difunkioneller Carbonsäuren. Als Carbonsäuren eignen sich hierfür solche mit Kohlenstoffketten von 2 bis 20 C-Atomen, vorzugsweise von 5 bis 15 C-Atomen. Gut geeignet sind auch Allyl- oder Vinylester aromatischer Dicarbonsäuren, wie Phthalsäure oder Trimellithsäure. Gut geeignet sind auch Allylether polyfunktioneller Alkohole, bevorzugt mindestens trifunktioneller Alkohole. Als Beispiel genannt seien die Allylether des Trimethylpropans, Pentaerythrittriallylether oder 2,2-Bis-oxyphenylpropan-bis-(diallylphosphat). Gut geeignet sind auch Verbindungen des Typs Cyanursäuretriallylester, Triallyltriazintrion und dergleichen.

Da die erfindungsgemäß einzusetzenden Dendrimere bereits mit C-C-Doppelbindungen terminiert sind, kann die Komponente (A) entfallen aber auch zu einem hohen Anteil enthalten sein. Je nach gewünschten Eigenschaften der Dentalmassen liegt die Komponente (H) in Mengen von 0,1 bis 50 Gew.-%, vorzugsweise 0,1 bis 20 Gew.-%, vor. Bereits geringste Zugaben bewirken eine erhebliche Erhöhung der Endhärte von Abformmassen.

Die Massen werden hergestellt durch Vermischen der Komponenten (A) bis (H) und härten in einer als Hydrosilylierung bezeichneten Additionsreaktion aus, bei der sich unter dem Einfluß des Platinkatalysators (D) die SiH-Gruppen der Komponente (B) an die ungesättigten Gruppen der Komponenten (A) bzw. (H) addieren. Aus Lagerstabilitätsgründen ist es bevorzugt, die Massen in einer zweikomponentigen Darreichungsform zu formulieren, bei der die gesamte Komponente (B) in einer sogenannten Basispaste untergebracht ist. Räumlich getrennt hiervon wird die gesamte Komponente (D) in einer sogenannten Katalysatorpaste untergebracht. Die Komponenten (A) bzw. (H) können entweder in der Katalysator- oder Basispaste untergebracht sein, wobei bevorzugt je ein Teil der Komponenten (A) bzw. (H) in der Basis- und ein Teil der Komponente (A) bzw. (H) in der Katalysatorpaste untergebracht sind. Die Komponenten (C), (E), (F) und (G) können in ihrer Gesamtmenge in der Katalysator- oder in der Basispaste untergebracht sein, wobei bevorzugt ist, daß jeweils ein Teil der jeweiligen Komponente in der Katalysator- und ein Teil in der Basispaste untergebracht sind.

Die Volumenverhältnisse von Katalysator- und Basispaste können 10:1 bis 1:10 betragen. Besonders bevorzugte Volumenverhältnisse von Basis- : Katalysatorpaste sind 1:1 und 5:1 (5 Teile Basis- : 1 Teil Katalysatorpaste). Im Falle eines Volumenverhältnisses von 1:1 können die Komponenten (A) bis (H) wie folgt auf Basis- und Katalysatorpaste verteilt sein.

**Tabelle 1**

| Komponente | Basispaste (Gew.-%) | Katalysatorpaste (Gew.-%) | Summe in Basis- und Katalysatorpaste (Gew.-%) |
|---|---|---|---|
| (A) | 0-60 | 0-60 | 0-60 |
| (B) | 2-60 | - | 1-30 |
| (C) | 0-20 | 0-20 | 0-20 |
| (D) | - | 0,0001 -0,1 | 0,00005-0,05 |
| (E) | 0-10 | 0-10 | 0-10 |
| (F) | 10-90 | 10-90 | 10-90 |
| (G) | 0-4 | 0-4 | 0-2 |
| (H) | 0-50 | 0-50 | 0,1-50 |

Im Falle eines Volumenverhältnisses von 5 Teilen Basispaste zu 1 Teil Katalysatorpaste können bevorzugte Mengenanteile wie folgt wiedergegeben werden:

**Tabelle 2**

| Komponente | Basispaste (Gew.-%) | Katalysatorpaste (Gew.-%) | Summe in Basis- und Katalysatorpaste (Gew.-%) |
|---|---|---|---|
| (A) | 0-60 | 0-60 | 0-60 |
| (B) | 1,2-36 | - | 1-30 |
| (C) | 0-24 | 0-20 | 0-20 |
| (D) | - | 0,00025-0,25 | 0,00005-0,05 |
| (E) | 0-10 | 0-10 | 0-10 |
| (F) | 10-90 | 5-90 | 10-90 |
| (G) | 0-2,4 | 0-4 | 0-2 |
| (H) | 0-50 | 0-50 | 0,1-50 |

Bei einem Volumenverhältnis 5:1 können die beiden Pasten in Schlauchfolienbeutel abgefüllt und später kurz vor der Verwendung mit Hilfe des Misch- und Dosiergerätes PENTAMIX® (Fa. ESPE) angemischt werden.

Möglich ist auch eine Darreichung in Form von handelsüblichen Doppelkammerkartuschen oder Kapseln.

Die Massen eignen sich insbesondere als Dentalmaterialien und zeichnen sich durch eine außergewöhnlich gute Endhärte von vorzugsweise Shorehärte A ≥ 80, bevorzugt ≥ 90, besonders bevorzugt Shorehärte D ≥ 40 bei ausgezeichneter Verarbeitungsviskosität aus. Als Dentalmaterialien kommen insbesondere Massen für die Bißregistrierung, provisorische und permanente Füllungsmaterialien, Kronen- und Brückenmaterialien sowie Zemente und Lacke in Betracht.

### Beispiele

### Herstellungsbeispiel

### 1. Chlor-Vorstufe

In einem 50 ml Dreihalskolben werden unter Stickstoff 2 g Tetravinylsilan und 5 Tropfen Karstedt-Katalysator vorgelegt. Bei Raumtemperatur tropft man 12,1 g Trichlorsilan zu und erhitzt anschließend noch 9 h auf 60°C. Flüchtige Bestandteile werden im Vakuum abdestilliert. Man erhält 10 g eines hellgelben Öls.

### 2. Allyl-Verbindung

10 g der Chlor-Vorstufe aus Stufe 1 werden in einem 1 1-Dreihalskolben mit Rückflußkühler und Tropftrichter unter Stickstoff vorgelegt. Bei Raumtemperatur werden 800 ml einer Lösung von Allylmagnesiumbromid in Diethylether (1 Mol/l) zugetropft, das Reaktionsgemisch kommt dabei zum Sieden. Anschließend erhitzt man noch 36 h unter Rückfluß. Das abgekühlte Gemisch wird in Portionen auf eiskalte, gesättigte Ammoniumchloridlösung gegeben, das Produkt mit Ether extrahiert und über Magnesiumsulfat getrocknet. Nach Abdestillieren des Lösungsmittels und säulenchromatographischer Reinigung (Kieselgel, Eluent Hexan/Trichlormethan 5 : 1) erhält man 2,3 g (29 % d. Th.) einer farblosen Allylverbindung.

### Anwendungsbeispiel

Dentale Abdruckmassen werden bei Verwendung von Platinkatalysatoren beispielsweise zweikomponentig im Verhältnis 1 : 1 formuliert. Nachfolgend wird daher die Formulierung einer Basis- und einer Katalysatorpaste angegeben.

### 1. Basispaste

23 Gew.-% Vinylsiloxan mit einer Viskosität von 200 mPa·s (Komponente (A)), 3 Gew.-% eines Polydimethylhydrogensiloxans mit einer Viskosität von 30 mPa·s und einem SiH-Gehalt von 7,3 mMol/g (Komponente (B)) und 1 Gew.-% eines Polydimethylsiloxans mit einer Viskosität von 50 mPa·s (Komponente (G)) werden eine Stunde unter Vakuum verrührt. Anschließend werden 72 Gew.-% feinstgemahlenem oberflächenbehandeltem Quarz (Komponente (F)) und 1 Gew.-% Dendrimer aus Herstellungsbeispiel 2 (Komponente (H)) eine Stunde unter Vakuum zugeknetet.

### 2. Katalysatorpaste

28 Gew.-% Vinylsiloxan mit einer Viskosität von 200 mPa·s und 1 Gew.-% eines Polydimethylsiloxans mit einer Viskosität von 50 mPa·s werden eine Stunde unter Vakuum verrührt. Nach der Zugabe von 70,5 Gew.-% feingemahlenem Quarz, der nicht oberflächenbehandelt wurde, sowie 0,5 Gew.-% Platin-Katalysator (1,3 % Platinanteil in Silikonöl, Komponente (D)) wurde eine Stunde ohne Vakuum geknetet.

### 3. Basispaste ohne Dendrimer

Anstelle von 1 Gew.-% Dendrimer werden ingesamt 24 Gew.-% Vinylsiloxan mit 200 mPa·s eingesetzt.

### Meßergebnisse

Gleiche Anteile Basispaste 1 bzw. 3 und Katalysatorpaste 2 werden auf einem Anmischblock mittels eines Spatels homogen vermischt und die Abbindezeit sowie die Shorehärte D (DIN 53505) auf übliche Weise gemessen.

| Basispaste | Abbindung bei 23°C (sec.) | Shorehärte D nach 24 h |
|---|---|---|
| 1 | 35-75 | 41 |
| 3 | 30-70 | 35 |

Der beschriebene erfindungsgemäße Effekt, die Erhöhung der Endhärte, ist deutlich zu erkennen. Der meßbare Parameter der Shorehärte nimmt um 6 Einheiten zu, wenn erfindungsgemäß ein Silan-Dendrimer eingesetzt wird.

## Patentansprüche

1. Härtbare Masse, enthaltend
(A) gegebenenfalls Organopolysiloxane mit mindestens zwei ungesättigten Gruppen im Molekül,
(B) Organohydrogenpolysiloxane mit mindestens 3 SiH-Gruppen im Molekül,
(C) gegebenenfalls Organopolysiloxane ohne reaktive Gruppen,
(D) Katalysator,
(E) gegebenenfalls Hydrophilierungsmittel,
(F) Füllstoffe und
(G) gegebenenfalls übliche dentale Zusatz-, Hilfs- und Farbstoffe,
**dadurch gekennzeichnet, daß** sie zusätzlich
(H) mindestens ein Silan-Dendrimer mit terminalen Alkenylgruppen enthält.

2. Masse nach Anspruch 1, enthaltend
5-70 Gew.-% Komponenten (A) + (B) + (H)
0-40 Gew.-% Komponente (C)
0,00005-0,05 Gew.-% Komponente (D), bezogen auf elementares Platin
0-10 Gew.-% Komponente (E)
10-90 Gew.-% Komponente (F)
0-2 Gew.-% Komponente (G),
0,1-50 Gew.-% Komponente (H).

3. Masse nach den Ansprüchen 1 und 2, enthaltend
10-60 Gew.-% Komponenten (A) + (B) + (H)
0-20 Gew.-% Komponente (C)
0,0002-0,04 Gew.-% Komponente (D), bezogen auf elementares Platin
0-2 Gew.-% Komponente (E)
30-80 Gew.-% Komponente (F)
0-1 Gew.-% Komponente (G)
0,1-20 Gew.-% Komponente (H).

4. Masse nach den Ansprüchen 1 bis 3, **dadurch gekennzeichnet, daß** das Mengenverhältnis der Komponenten (A) + (H) zu Komponente (B) so gewählt wird, daß pro Mol an ungesättigter Doppelbindung der Komponenten (A) und (H) 0,5 bis 10 Mol SiH-Gruppen der Komponente (B) vorliegen.

5. Masse nach den Ansprüchen 1 bis 4, **dadurch gekennzeichnet, daß** sie als Komponente (H) ein Dendrimeres der folgenden Formel enthält:
SiRₙR'₄₋ₙ
in welcher bedeuten:
R eine Alkenylgruppe mit 2 bis 5 C-Atomen, wobei die C-C-Doppelbindung terminal vorliegt, vorzugsweise eine Vinyloder Allylgruppe,
n eine ganze Zahl von 1 bis 4 und
R' einen verzweigten oder unverzweigten aliphatischen oder aromatischen Kohlenwasserstoffrest mit 1 bis 10 C-Atomen oder Wasserstoff, vorzugsweise Methyl oder Phenyl.

6. Masse nach den Ansprüchen 1 bis 4, **dadurch gekennzeichnet, daß** sie als Komponente (H) ein Dendrimeres der folgenden Formel enthält:
SiR'ₓ((CH₂)ₙ-Si-((CH₂)ₘ-CH=CH₂)₃)₄₋ₓ
in welcher bedeuten:
R' wie in Anspruch 5 definiert,
n = 2, 3, 4 oder 5,
m = 0, 1, 2 oder 3, und
x = 0 oder 1.

7. Masse nach Anspruch 6, **dadurch gekennzeichnet, daß** sie als Komponente (H) mindestens eines der folgenden Dendrimere enthält:
Me-Si((CH₂-CH₂-Si(Vinyl)₃)₃
Si((CH₂-CH₂-Si(Vinyl)₃)₄
Me-Si((CH₂-CH₂-CH₂-Si(Allyl)₃)₃
Si((CH₂-CH₂-CH₂-Si(Allyl)₃)₄
Me-Si((CH₂-CH₂-Si(Allyl)₃)₃
Si((CH₂-CH₂-Si(Allyl)₃)₄
Me-Si((CH₂-CH₂-CH₂-Si(Vinyl)₃)₃
Si((CH₂-CH₂-CH₂-Si(Vinyl)₃)₄.

8. Masse nach den Ansprüchen 1 bis 4, **dadurch gekennzeichnet, daß** sie als Komponente (H) ein Dendrimeres der folgenden Formeln enthält: 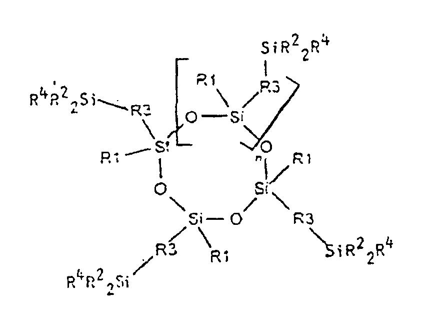 in welcher bedeuten:
n = 0, 1, 2, 3, 4 oder 5,
R¹ einen gesättigten Alkylrest mit 1 bis 6 C-Atomen oder Wasserstoff,
R² eine Alkenylgruppe mit 2 bis 6 C-Atomen, wobei die C-C-Doppelbindung terminal vorliegt,
R³ eine Alkylengruppe mit 2 bis 6 C-Atomen und
R⁴ entweder R² oder -O-SiMe₂-Allyl oder -O-SiMe₂-Vinyl.

9. Masse nach Anspruch 8, **dadurch gekennzeichnet, daß**
n = 0,
R¹ = CH₃ und
R² = Allyl oder Vinyl bedeuten.

10. Masse nach den Ansprüchen 1 bis 9, **dadurch gekennzeichnet, daß** sie in Form einer Basispaste und einer davon räumlich getrennten Katalysatorpaste vorliegt, wobei die gesamte Komponente (B) in der Basispaste und die gesamte Komponente (D) in der Katalysatorpaste vorhanden sind und die übrigen Komponenten wahlweise in den beiden Pasten verteilt sind.

11. Masse nach den Ansprüchen 1 bis 10, **dadurch gekennzeichnet, daß** das Volumenverhältnis von Basispaste zu Katalysatorpaste 10 : 1 bis 1 : 10, vorzugsweise 1 : 1 oder 5 : 1, beträgt.

12. Verwendung einer härtbaren Masse, enthaltend die Komponenten
(A) gegebenenfalls Organopolysiloxane mit mindestens zwei ungesättigten Gruppen im Molekül,
(B) Organohydrogenpolysiloxane mit mindestens 2 SiH-Gruppen im Molekül,
(C) gegebenenfalls Organopolysiloxane ohne reaktive Gruppen,
(D) Katalysator,
(E) gegebenenfalls Hydrophilierungsmittel,
(F) Füllstoffe,
(G) gegebenenfalls weitere übliche Zusatz-, Hilfs- und Farbstoffe, und
(H) mindestens ein Silan-Dendrimer mit terminalen Alkenylgruppen
als Dentalmasse, insbesondere als Massen für die Bißregistrierung, provisorische und permanente Füllungsmaterialien, Kronen- und Brückenmaterialien sowie Zemente und Lacke.

## Claims

1. Curable material containing
(A) optionally organopolysiloxanes with at least two unsaturated groups in the molecule,
(B) organohydrogenpolysiloxanes with at least 3 SiH groups in the molecule,
(C) optionally organopolysiloxanes without reactive groups,
(D) catalyst
(E) optionally hydrophilizing agents,
(F) fillers and
(G) optionally conventional dental additives, adjuvants and colorants,
**characterized in that** they additionally
(H) contain at least one silane dendrimer with terminal alkenyl groups.

2. Material according to claim 1 containing
5 - 70 wt.-% components (A) + (B) + (H)
0 - 40 wt.-% component (C)
0.00005 - 0.05 wt.-% component (D), in relation to elemental platinum
0 - 10 wt.-% component (E)
10 - 90 wt.-% component (F)
0 - 2 wt.-% component (G)
0.1 - 50 wt.-% component (H).

3. Material according to claims 1 and 2, containing
10 - 60 wt.-% components (A) + (B) + (H)
0 - 20 wt.-% component (C)
0.0002 - 0.04 wt.-% component (D), relative to elementary platinum
0 - 2 wt.-% component (E)
30 - 80 wt.-% component (F)
0 - 1 wt.-% component (G)
0.1 - 20 wt.-% component (H).

4. Material according to claims 1 to 3, **characterized in that** the quantity ratio of components (A) + (H) to component (B) are chosen so that 0.5 to 10 mol SiH groups of component (B) are present per mol of unsaturated double bond of components (A) and (H).

5. Material according to claims 1 to 4, **characterized in that** it contains a dendrimer of the following formula as component (H):
SiRₙR'₄₋ₙ
in which:
R is an alkenyl group with 2 to 5 C atoms, the C-C double bond being terminally present, preferably a vinyl or allyl group,
n is an integer from 1 to 4 and
R' is a linear or branched aliphatic or aromatic hydrocarbon radical with 1 to 10 C atoms or hydrogen, preferably methyl or phenyl.

6. Material according to claims 1 to 4, **characterized in that** it contains a dendrimer of the following formula as component (H):
SiR'ₓ((CH₂)ₙ-Si((CH₂)ₘ-CH=CH₂)₃)₄₋ₓ
in which:
R' is defined as in claim 5,
n is 2, 3, 4 or 5,
m is 0, 1, 2 or 3, and
x means 0 or 1.

7. Material according to claim 6, **characterized in that** it contains at least one of the following dendrimers as component (H):
Me-Si((CH₂-CH₂-Si(vinyl)₃)₃
Si((CH₂-CH₂-Si(vinyl)₃)₄
Me-Si((CH₂-CH₂-CH₂-Si)allyl)₃)₃
Si-((CH₂-CH₂=-CH₂-Si(allyl) ₃)₄
Me-Si((CH₂-CH₂-Si(allyl)₃)₃
Si((CH₂-CH₂-Si(allyl)₃)₄
Me-Si((CH₂-CH₂-CH₂-Si(vinyl)₃)₃
Si((CH₂-CH₂-CH₂-Si(vinyl)₃)₄.

8. Material according to claims 1 to 4, **characterized in that** it contains a dendrimer of the following formula as component (H): 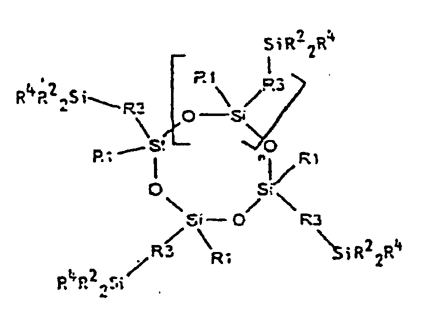 in which:
n is 0, 1, 2, 3, 4 or 5,
R' is a saturated alkyl radical with 1 to 6 C atoms or hydrogen,
R² is an alkenyl group with 2 to 6 C atoms, the C-C double bond being terminally present,
R³ is an alkenyl group with 2 to 6 C atoms and
R⁴ is either R² or -O-SiMe₂-allyl or -O-SiMe₂-vinyl.

9. Material according to claim 8, **characterized in that** n is 0,
R¹ is CH₃ and
R² is allyl or vinyl.

10. Material according to claims 1 to 9, **characterized in that** it is present in the form of a base paste and a catalyst paste physically separated from it, the whole component (B) being present in the base paste and the whole component (D) being present in the catalyst paste and the remaining components being optionally distributed in the two pastes.

11. Material according to claims 1 to 10, **characterized in that** the volume ratio of base paste to catalyst paste is 10 : 1 to 1 : 10, preferably 1 : 1 or 5 : 1.

12. Use of a curable material containing the components
(A) optionally organopolysiloxanes with at least two unsaturated groups in the molecule,
(B) organohydrogenpolysiloxanes with at least 2 SiH groups in the molecule,
(C) optionally organopolysiloxanes without reactive groups
(D) catalyst
(E) optionally hydrophilizing agents,
(F) fillers
(G) optionally other conventional additives, adjuvants and colorants, and
(H) at least one silane dendrimer with terminal alkenyl groups
as dental material, in particular as materials for bite registration, temporary or permanent filling materials, crown and bridging materials as well as cements and enamel.

## Revendications

1. Masse durcissable contenant
(A) le cas échéant des organopolysiloxanes avec au moins deux groupes insaturés dans la molécule,
(B) des organohydrogénopolysiloxanes avec au moins 3 groupes SiH dans la molécule,
(C) le cas échéant des organopolysiloxanes sans groupes réactifs,
(D) un catalyseur,
(E) le cas échéant des produits hydrophiles,
(F) des matières de charge et
(G) le cas échéant les additifs, auxiliaires et colorants dentaires habituels,
**caractérisée en ce qu'**elle contient en plus
(H) au moins un dendrimère de silane avec des groupes alkényles terminaux.

2. Masse selon la revendication 1, contenant
5 à 70 % en poids des composants (A) + (B) + (H)
0 à 40 % en poids du composant (C)
0,00005 à 0,05 % en poids du composant (D), extrait de platine élémentaire
0 à 10 % en poids du composant (E)
10 à 90 % en poids du composant (F)
0 à 2 % en poids du composant (G),
0,5 à 50 % du composant (H).

3. Masse selon les revendications 1 et 2, contenant
10 à 60 % en poids des composants (A) + (B) + (H)
0 à 20 % en poids du composant (C)
0,0002 à 0,04 % en poids du composant (D) extrait de platine élémentaire
0 à 2 % en poids du composant (E)
30 à 80 % en poids du composant (F)
0 à 1 % en poids du composant (G)
0,1 à 20 % en poids du composant (H)

4. Masse selon les revendications 1 à 3, **caractérisée en ce que** le rapport quantitatif des composants (A) + (H) par rapport au composant (B) est choisi pour que par mole de liaisons doubles insaturées des composants (A) et (H), 0,5 à 10 moles du composant (B) sont disponibles.

5. Masse selon les revendications 1 à 4, **caractérisée en ce qu'**elle contient comme composant (H) un dendrimère de la formule suivante :
SiRₙR'₄₋ₙ
dans laquelle :
R signifie un groupe alkyle avec 2 à 5 atomes de carbone, la liaison double C-C étant disponible en fin de chaîne, de préférence un groupe vinyle ou allyle,
H signifie un nombre entier de 1 à 4 et
R' signifie un radical hydrocarbure aliphatique ou aromatique ramifié ou non-ramifié avec 1 à 10 atomes de carbone ou un hydrogène, de préférence un méthyle ou un phényle.

6. Masse selon les revendications 1 à 4, **caractérisée en ce qu'**elle contient comme composant (H) un dendrimère de la formule suivante :
SiR'ₓ((CH₂)ₙ-Si-((CH₂)ₘ-CH=CH₂)₃)₄₋ₓ
dans laquelle :
R' est comme défini dans la revendication 5,
n = 2, 3, 4 ou 5,
m = 0, 1, 2 ou 3, et
x = 0 ou 1.

7. Masse selon la revendication 6, **caractérisée en ce qu'**elle contient comme composant (H) au moins un des dendrimères suivants :
Me-Si((CH₂-CH₂-Si(vinyle)₃)₃
Si((CH₂-CH₂-Si-(vinyle)₃)₄
Me-Si((CH₂-CH₂-CH₂-Si(allyle)₃)₃
Si((CH₂-CH₂-CH₂-Si(allyle)₃)₄
Me-Si((CH₂-CH₂-Si(allyle)₃)₃
Si((CH₂-CH₂-Si(allyle)₃)₄
Me-Si((CH₂-CH₂-CH₂-Si(vinyle)₃)₃
Si((CH₂-CH₂-CH₂-Si(vinyle)₃)₄.

8. Masse selon les revendications 1 à 4, **caractérisée en ce qu'**elle contient comme composant (H) un dendrimère de la formule suivante : 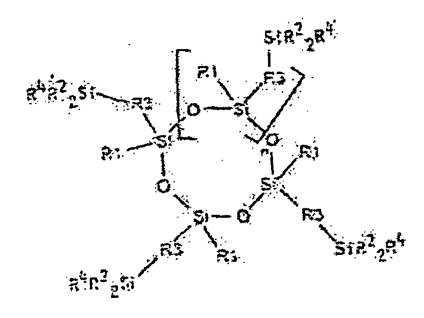 dans laquelle signifient :
n = 0, 1, 2, 3, 4.ou 5,
R¹ un radical alkyle saturé avec 1 à 6 atomes de carbone, ou un hydrogène,
R₂ un groupe alkyle avec 2 à 6 atomes de carbone, la double liaison C-C étant disponible en fin de chaîne,
R³ un groupe alkyle avec 2 à 6 atomes de carbones et
R⁴ soit R², soit -O-SiMe₂-allyle ou -O-SiMe₂-Vinyle.

9. Masse selon la revendication 8, **caractérisée en ce que**
n = 0,
R¹ = CH₃ et
R² = allyle ou vinyle.

10. Masse selon les revendications 1 à 9, **caractérisée en ce qu'**elle est disponible sous la forme d'une pâte de base et d'une pâte catalyseuse séparée dans l'espace, tout le composant (B) se trouvant dans la pâte de base et tout le composant (D) dans la pâte catalyseuse et les composants restants étant dispersés au choix dans les deux pâtes.

11. Masse selon les revendications 1 à 10, **caractérisée en ce** le rapport volumique de la pâte de base par rapport à la pâte catalyseuse s'élève de 10:1 à 1:10, de préférence 1:1 ou 5:1.

12. Utilisation d'une masse durcissable, contenant les composants
(A) le cas échéant des organopolysiloxanes avec au moins deux groupes insaturés dans la molécule,
(B) des organohydrogénopolysiloxanes avec au moins 2 groupes SiH dans la molécule,
(C) le cas échéant des organopolysiloxanes sans groupes réactifs,
(D) un catalyseur,
(E) le cas échéant des produits hydrophiles,
(F) des matières de charge,
(G) le cas échéant d'autres additifs, auxiliaires et colorants habituels et
(H) au moins un dendrimère de silane avec des groupes alkényles terminaux
comme masse dentaire, en particulier comme empreintes dentaires, les amalgames provisoires ou permanents, les matériaux pour couronnes et bridges, ainsi que des ciments et des laques.
